(19) Europäisches Patentamt
European Patent Office
Office européen des brevets

(11) Veröffentlichungsnummer: **0 014 261**
**B1**

(12) # EUROPÄISCHE PATENTSCHRIFT

(45) Veröffentlichungstag der Patentschrift: **07.07.82**

(51) Int. Cl.³: **C 07 D 209/88,** C 09 B 49/10

(21) Anmeldenummer: **79105386.1**

(22) Anmeldetag: **27.12.79**

(54) **Verfahren zur Herstellung von Derivaten des 3-(4-Oxy-anilino)-carbazols und Verwendung der nach diesem Verfahren hergestellten Derivate zur Herstellung von Schwefelfarbstoffen.**

(30) Priorität: **08.01.79 DE 2900441**

(43) Veröffentlichungstag der Anmeldung: **20.08.80 Patentblatt 80/17**

(45) Bekanntmachung des Hinweises auf die Patenterteilung: **07.07.82 Patentblatt 82/27**

(84) Benannte Vertragsstaaten: **CH DE FR GB IT NL**

(56) Entgegenhaltungen: **DE-A 1227 323**

(73) Patentinhaber: **CASSELLA Aktiengesellschaft, Hanauer Landstrasse 526, D-6000 Frankfurt am Main 61 (DE)**

(72) Erfinder: **Piesch, Steffen, Dr., An der Heide 32, D-6370 Oberursel/Taunus 1 (DE)**
Erfinder: **Engelhardt, Friedrich, Dr., Hünfelder Strasse 20, D-6000 Frankfurt am Main 61 (DE)**
Erfinder: **Wille, Herbert, Dr., Hünfelder Strasse 16, D-6000 Frankfurt am Main 61 (DE)**
Erfinder: **Weidemüller, Wolf, Dr., Nordring 101, D-6000 Frankfurt am Main 60 (DE)**
Erfinder: **Meyer, Artur, Dr., Weinbergstrasse 6, D-6369 Schöneck 1 (DE)**

(74) Vertreter: **Urbach, Hans-Georg, Dr., Hanauer Landstrasse 526, D-6000 Frankfurt am Main 61 (DE)**

**EP 0 014 261 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Derivaten des 3-(4-Oxy-anilino)-carbazols und Verwendung der nach diesem Verfahren hergestellten Derivate zur Herstellung von Schwefelfarbstoffen

Die Erfindung betrifft ein Verfahren zur Herstellung von Derivaten des 3-(Oxy-anilino)-carbazols der allgemeinen Formel

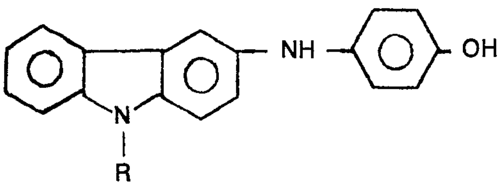

I

worin R = Wasserstoff, Alkyl mit 1 bis 8 C-Atomen, Halogenalkyl mit 1 bis 8 C-Atomen bedeutet und die Verwendung der nach diesem Verfahren hergestellten Derivate zur Herstellung von Schwefelfarbstoffen.

Die Verbindungen der allgemeinen Formel I werden unter anderem auch als Carbazolyl-3--aminophenole oder als Leucoverbindungen von Carbazol-indophenolen bezeichnet. Die Carbazol-indophenole und die Verbindungen der allgemeinen Formel I sind unentbehrliche Ausgangsmaterialien zur Herstellung wertvoller Schwefelfarbstoffe vorwiegend blauer Nuance. Die Verbindungen der Formel I werden bis jetzt durch Reduktion der entsprechenden Carbazol--indophenole (Carbazolyl-3-iminochinone bzw. Benzochinon(1,4)-monocarbazolyl-(3)-imide) hergestellt, für deren Herstellung bis jetzt im wesentlichen zwei Verfahren bekannt geworden sind:

1. Durch Kondensation von Carbazol bzw. von N-substituierten Carbazolen mit p-Nitrosophenol in kalter, hochprozentiger Schwefelsäure unter Kühlung (FR-PS 457 535, deutsche Reichspatentschriften 218 371, 224 951 und 230 119).

2. Durch Umsetzung von Carbazol und seiner Derivate mit p-Aminophenol in konzentrierter Schwefelsäure unter Kühlung in Gegenwart eines Oxidationsmittels, wie Mangandioxid (FR-PS 457 535).

Die anschliessende Reduktion der Carbazol--indophenole zu den Verbindungen der allgemeinen Formel I erfolgt im allgemeinen in verdünnter Säure mit Eisen.

3. Zur Herstellung bestimmter Derivate der Verbindungen der allgemeinen Formel I ist es auch bekannt (FR-PS 390 715), bestimmte Derivate des 2,2'-Dinitrophenyls zu reduzieren und durch Behandlung mit verdünnten, kochenden Mineralsäuren einen Ringschluss zum Carbazolderivat herbeizuführen.

In O. Lange «Die Zwischenprodukte der Teerfarbenfabrikation», Leipzig 1920, wird auf Seite 315 auf die deutsche Patentanmeldung A 25 495, Klasse 12 P, vom 24.2.1914 hingewiesen, in der die Herstellung der Indophenole und ihrer Leuco-Verbindungen aus 3-Aminocarbazol oder seinen N-substituierten Derivaten durch Kondensation mit Hydrochinon in Gegenwart wasserentziehender Mittel unter Vermeidung der Oxidation beschrieben sein soll. Die Anmeldung wurde nicht veröffentlicht, so dass Details dieses Verfahrens nicht bekannt sind. Bei der Durchführung der Reaktion in Gegenwart der damals bekannten wasserentziehenden Mittel, nämlich konzentrierter Schwefelsäure oder Zinkchlorid, ist die Aufarbeitung durch Wasser zwingend notwendig, und man erhält das Leuco-indophenol nur in geringer Ausbeute neben einer Reihe von unerwünschten Nebenprodukten. Die Reinigung der so erhaltenen Leuco-indophenole gestaltet sich schwierig und schliesst die Anwendung dieses Verfahrens für die technische Herstellung der gewünschten Produkte aus. Auch die oben unter 2. und 3. genannten bekannten Herstellungsverfahren liefern die gewünschten Indophenole bzw. Leuco-indophenol nicht in der gewünschten Ausbeute und Reinheit und konnten im Laufe der langen Zeit, seit der sie bekannt wurden, nicht soweit verbessert werden, dass sie technisch durchführbar sind. Als optimales bekanntes Verfahren hat sich bisher in der Technik das oben unter 1. genannte Kondensationsverfahren behauptet, bei dem zunächst p-Nitrosophenol mit dem Carbazolderivat kondensiert wird und das erhaltene chinoide Zwischenprodukt des Indophenols anschliessend in der Regel reduziert werden muss, da das chinoide Produkt besonders instabil ist.

Dieses bekannte Verfahren hat gravierende technische Nachteile. Einerseits werden die Verfahrensprodukte nicht in gleichmässiger Qualität erhalten. Vielmehr schwankt die Qualität der erhaltenen Indophenole aufgrund nicht genau kontrollierbarer geringfügiger Verfahrensabweichungen oder geringfügiger Abweichungen in der Qualität der Ausgangsmaterialien oder des zur Reduktion verwendeten Eisens, was sich in unterschiedlichem Ausfall der Qualität der aus den Indophenolen hergestellten Schwefelfarbstoffe bemerkbar macht. Ausserdem werden für die Durchführung dieses Verfahrens grosse Mengen von Schwefelsäure benötigt, die heute entweder wieder aufgearbeitet oder in anderer ökologisch einwandfreier Weise beseitigt werden müssen. Auch die Herstellung des als Ausgangsmaterial für dieses Verfahren benötigten p-Nitrosophenols ist mit erheblichen ökologisch bedingten Schwierigkeiten verbunden. Hinzu kommt, dass p-Nitrosophenol thermisch instabil ist und seine Qualität sich mit der Lagerzeit verändert.

Trotz aller genannten Schwierigkeiten der bisher bekannten Verfahren und trotz der jahrzehntelangen intensiven Suche nach Verfahrensverbesserungen ist bisher die Kondensation von Carbazolen mit p-Nitrosophenol als das bisher optimale technische Herstellungsverfahren für die gewünschten Indophenole und Leuco-indophenole angesehen worden. Jetzt wurde überraschenderweise gefunden, dass man die Nachteile und Schwierigkeiten der bisher bekannten Verfahren zur Herstellung von Leuco-indopheno-

len der allgemeinen Formel I vermeiden kann und diese Produkte in einfacher Weise und in sehr guter Ausbeute und hoher Reinheit herstellen kann.

Das erfindungsgemässe Verfahren zur Herstellung von Verbindungen der allgemeinen Formel I ist dadurch gekennzeichnet, dass ein 3-Aminocarbazol der allgemeinen Formel

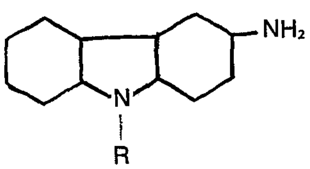

II

mit p-Hydrochinon oder p-Aminophenol oder mit einem Gemisch aus p-Hydrochinon und p-Aminophenol in mindestens stöchiometrischen Mengen unter Ausschluss von Sauerstoff auf Temperaturen von 150 bis 300°C, zweckmässigerweise auf 170 bis 230°C, erhitzt wird. Bei der Durchführung des erfindungsgemässen Verfahrens werden im einfachsten Fall die Reaktionsteilnehmer zusammengeschmolzen, gegebenenfalls mit p-Hydrochinon oder p-Aminophenol als Überschuss und so lange auf der Kondensationstemperatur zwischen 150 und 250°C, vorzugsweise 170 bis 230°C, gehalten, bis kein Reaktionswasser oder Ammoniak aus dem Ansatz mehr abdestilliert. Je nach der gewählten Reaktionstemperatur ist die Reaktion in der Regel nach 2 bis 10 Stunden praktisch abgeschlossen. Die Aufarbeitung des Reaktionsgemisches erfolgt im einfachsten Fall durch Abdestillieren des Überschusses an p-Hydrochinon und/oder p-Aminophenol zweckmässigerweise unter vermindertem Druck und anschliessendes Ablassen der Schmelze aus dem Reaktionsgefäss und nachfolgendem Zerkleinern in einer geeigneten Mühle. Es ist jedoch auch möglich, zum Zwekke der Reinigung die entstandene Verbindung der Formel I zu destillieren, was direkt aus dem Reaktionsgefäss heraus geschehen kann. Diese Destillation wird zweckmässigerweise unter vermindertem Druck vorgenommen. Es war ausserordentlich überraschend, dass die Verbindungen der Formel I, die bisher als ausserordentlich empfindliche und leicht zusetzliche Verbindungen angesehen wurden, sich durch eine derartige Herstellung und Destillation auf einfachstem Wege reinigen lassen. Selbst bei Sumpftemperaturen von 300°C und mehr findet praktisch keine Zersetzung statt.

Die Kondensationsreaktion zwischen den Aminocarbazolen der Formel II und p-Hydrochinon oder p-Aminophenol kann durch einen Zusatz einer katalytischen Menge von Jod deutlich beschleunigt werden und führt zusätzlich zu reineren Produkten. Als katalytische Mengen kommen normalerweise Zusätze von 0,1 bis 4, vorzugsweise 0,1 bis 1 Gew.-%, bezogen auf das eingesetzte Carbazolderivat der allgemeinen Formel II in Betracht. Da der Jod-Zusatz weder bei der Aufarbeitung der erfindungsgemäss hergestellten Leuco-indophenole, noch bei deren Weiterverarbeitung stört, ist die Durchführung des erfindungsgemässen Verfahrens unter Zusatz von katalytischen Mengen Jod bevorzugt.

Die erfindungsgemässe Kondensation des p-Hydrochinons und/oder p-Aminophenols mit dem Carbazolderivat der Formel II kann auch in Gegenwart eines inerten organischen Lösungsmittels durchgeführt werden. Zweckmässigerweise werden solche Lösungsmittel verwendet, deren Siedepunkt oberhalb der Kondensationstemperatur, d.h. insbesondere oberhalb von 150 bis 230°C liegt. Sofern aufgrund besonderer Überlegungen Lösungsmittel eingesetzt werden sollen, deren Siedepunkt tiefer als 150°C liegt, ist zweckmässigerweise in einem druckfest verschlossenen Gefäss (Autoklav) zu arbeiten. Organische Lösungsmittel, die für die Durchführung des erfindungsgemässen Verfahrens geeignet sind, sind z.B. Erdölfraktionen mit einem Siedepunkt über 150°C, cyclische Kohlenwasserstoffe, wie beispielsweise Decalin, oder aromatische Lösungsmittel, insbesondere das technische Gemisch aus Monochlor- und Dichlorbenzol. Insbesondere wenn die hergestellten Leuco-indophenole anschliessend in einer sogenannten Kochschmelze, d.h. einer Lösung von Natriumpolysulfiden, in Schwefelfarbstoffe überführt werden, kann die erfindungsgemässe Kondensation des p-Hydrochinons und/oder p-Aminophenols mit einem Carbazolderivat der allgemeinen Formel II auch in Gegenwart eines Lösungsmittels der allgemeinen Formel III oder IV

$R^1O\text{-}(CH_2CH_2O)_n\text{-}R^1$ (III)

$R^1O\text{-}(CH_2CH_2O)_n\text{-}H$ (IV)

worin $R^1 = -CH_3, -C_2H_5, -C_3H_7$ und $n = 1$, 2 oder 3 bedeuten, oder in einem höheren Alkohol oder Alkanoläther durchgeführt werden. Geeignete Lösungsmittel der allgemeinen Formeln III und IV sind z.B. Diglykoldimethyläther, Diglykoldiäthyläther, Glykoldiäthyläther, Äthylenglykol-mono-methyläther, -mono-äthyläther, -mono-propyläther, Diäthylenglykol-mono-methyläther, -mono-propyläther. Bei Verwendung von Lösungsmitteln der allgemeinen Formel III oder IV oder in einem höheren Alkohol oder Alkanoläther kann die rohe Reaktionsmischung des Leuco-indophenols direkt der Schwefelung unterworfen werden.

Wenn die Herstellung der Verbindungen der allgemeinen Formel I nach dem erfindungsgemässen Verfahren in dem bevorzugten Temperaturbereich von 170 bis 230°C in Gegenwart eines inerten Lösungsmittels durchgeführt wird, so sollte dieses bei Normaldruck zweckmässigerweise einen Siedepunkt von 180 bis 240°C, oder höher, besitzen. Zweckmässigerweise wird ein solches inertes Lösungsmittel ausgewählt, das mit Wasser bei Normaltemperatur nicht mischbar ist. Derartige inerte Lösungsmittel wirken bei der Umsetzung des Carbazolderivats II mit p-Hydrochinon als azeotrope Schleppmittel für das bei der Reaktion abgespaltene Wasser. Derartige als azeotrope Schleppmittel geeignete Lösungsmittel sind beispielsweise Decalin, Monochlor- und Dichlorbenzol.

Bei der Verwendung eines inerten Lösungsmittels wird dessen Menge so gering wie möglich gehalten. In der Regel ist es nicht erforderlich, mehr als 15 Gew.-% des inerten Lösungsmittels, bezogen auf das Gesamtgewicht der Reaktanten, einzusetzen.

Bei der Umsetzung der Verbindung der allgemeinen Formel II mit p-Hydrochinon und/oder p-Aminophenol wird der Ausschluss von Sauerstoff z.B. dadurch bewirkt, dass die Reaktion unter der Atmosphäre eines Inertgases, wie z.B. Stickstoff, durchgeführt wird. Insbesondere wenn kein inertes Lösungsmittel verwendet wird, ist es zweckmässig, mindestens die 1,3fach stöchiometrische Menge an p-Hydrochinon und/oder p-Aminophenol und vorzugsweise mindestens die 1,6fache stöchiometrische Menge an Hydrochinon und/oder p-Aminophenol einzusetzen. Das p-Aminophenol oder das p-Hydrochinon oder das Gemisch dieser Substanzen wirkt dabei als Lösungsmittel. Höhere Überschüsse an p-Hydrochinon und/oder p-Aminophenol als die 3fach stöchiometrische Menge brauchen normalerweise nicht eingesetzt zu werden. Nach beendeter Reaktion kann das überschüssige p-Aminophenol und/oder p-Hydrochinon leicht zweckmässig unter vermindertem Druck abdestilliert werden und kann in einem weiteren Ansatz wieder verwendet werden.

Bei der Durchführung der Reaktion zwischen dem Carbazolderivat der allgemeinen Formel II und dem p-Hydrochinon oder p-Aminophenol kann der Reaktionsverlauf entweder dünnschichtchromatographisch oder bei Verwendung von p-Hydrochinon durch Bestimmung des abgespaltenen Wassers oder bei Verwendung von p-Aminophenol durch titrimetrische Bestimmung des abgespaltenen Ammoniaks verfolgt werden.

p-Hydrochinon und p-Aminophenol sind bekannte Substanzen. Auch die 3-Aminocarbazole der allgemeinen Formel II sind zum grössten Teil bekannt. Die Verbindungen der allgemeinen Formel II mit R = Alkyl können leicht beispielsweise dadurch erhalten werden, dass Carbazol am Stickstoff, z.B. mit Dialkylsulfaten oder Alkylhalogeniden alkyliert wird und anschliessend in 3-Stellung eine Nitrogruppe durch Nitrierung eingeführt wird, die dann in bekannter Weise zur $-NH_2$-Gruppe reduziert wird.

Die in den erfindungsgemäss herstellbaren Leuco-indophenolen der Formel I enthaltenen Alkylreste R können linear oder verzweigt oder auch durch Halogen substituiert sein. Beispiele für Alkylreste R sind Methyl; Äthyl; Propyl-1 oder -2; Butyl-1 oder -2; 2-Methyl-propyl-1 oder -2; Pentyl-1, -2 oder -3; 2-Methyl-butyl-1, -2 oder -3; Hexyl-1, -2 oder -3; 2- oder 3-Methyl-hexyl-1, -2 oder -3; 2-Äthyl-butyl-1, -2 oder -3; Heptyl, Isoheptyl, Octyl, 2-Äthyl-hexyl-1, -2 oder -3; 3-Äthyl-hexyl-1, -2 oder -3; β-Chloräthyl; γ-Chlorpropyl.

Vorzugsweise dient das Verfahren der vorliegenden Erfindung zur Herstellung von Leuco-indophenolen der Formel I, bei denen R Wasserstoff oder Alkyl mit 1 bis 4 C-Atomen, insbesondere Äthyl, bedeutet.

Werden die erfindungsgemäss hergestellten Leuco-indophenole der Formel I durch nachfolgende Vakuum-Destillation gereinigt, so können daraus nach den bisher üblichen Schwefelungsverfahren Schwefelfarbstoffe hergestellt werden, die gegenüber entsprechenden Schwefelfarbstoffen, die aus herkömmlich gewonnenen Leuco-indophenolen der Formel I erhalten worden sind, erhöhte Brillanz und erhöhte Klarheit der Nuance aufweisen. In diesem Zusammenhang ist besonders hervorzuheben, dass Indophenole der Formel I, die nach bisher bekannten Verfahren, insbesondere durch Kondensation von Carbazolen mit p-Nitrosophenol in $H_2SO_4$ hergestellt worden sind, sich nicht unzersetzt destillieren lassen und ausserdem bei längerem Lagern bei mässig erhöhter Temperatur in steigendem Masse in nicht mehr schwefelbare Zersetzungsprodukte übergehen.

Die Vakuum-Destillation der erfindungsgemäss hergestellten Leuco-Indophenole bietet z.B. bei 0,1 bis 0,6 mbar keine technischen Schwierigkeiten. Das 3-(4-Oxy-anilino)-9-äthylcarbazol besitzt z.B. bei 0,25 bis 0,55 mbar einen Siedepunkt von 265 bis 282°C, und das 3-(4-Oxy-anilino)-carbazol besitzt z.B. bei 0,13 mbar einen Siedepunkt von 310 bis 320°C.

Das erfindungsgemässe Verfahren ist technisch einfach durchzuführen. Es arbeitet vor allem bei der bevorzugten Ausführung unter Zusatz von Jod mit ausgezeichneten Ausbeuten, insbesondere sehr guter Raum-Zeit-Ausbeute, es bedarf keiner umständlichen Isolierungsmassnahmen des Reaktionsproduktes, es entstehen praktisch keine Abwässer, Abfallösungsmittel oder sonstige ökologisch bedenkliche Abfall-Produkte, und es können vor allem bei der bevorzugten Ausführung unter Zusatz von Jod Produkte von bisher nicht erreichbarer Reinheit erhalten werden, die zur Herstellung von Schwefelfarbstoffen ausgezeichneter und stets reproduzierbarer Nuance und Farbeinheit dienen können. Im Hinblick auf die unterschiedliche Weiterverarbeitung der hergestellten Leuco-indophenole der Formel I ist die Durchführung des erfindungsgemässen Verfahrens ohne jedes Lösungsmittel am universellsten anwendbar.

Für die Weiterverarbeitung der erfindungsgemäss hergestellten Leuco-indophenole der Formel I nach bekannten Schwefelungsverfahren ist in der Regel eine Reinigung durch Destillation nicht erforderlich, sondern es kann direkt die Schmelze nach der Zerkleinerung den bekannten Schwefelungsoperationen unterworfen werden.

Die Schwefelung der erfindungsgemäss hergestellten Leuco-indophenole erfolgt in der Back- oder Kochschmelze in an sich bekannter Weise. Diese Schwefelungsverfahren sind beispielsweise in «Venkataraman, The Chemistry of Synthetic Dyes, Bd. 2 (1952), Seite 1062 ff. und Seite 1103 ff., sowie Bd. 7 (1974), Seite 24 ff. Academic Press, New York, San Francisco,

London», und in BIOS-Report 983, Seiten 70, 73-74, beschrieben.

Vorzugsweise erfolgt die Schwefelung der erfindungsgemäss hergestellten Leuco-indophenole nach dem Verfahren der Kochschmelze.

Beispiel 1

210 g 3-Amino-9-äthyl-carbazol (1 Mol), 220 g p-Hydrochinon (2 Mol), 2 g Jod werden unter Stickstoff bei 180 bis 200°C ca. 16 Stunden lang gerührt und das Reaktionswasser abdestilliert (ca. 18 ml). Dann wird bei einer Badtemperatur von 200 bis 220°C bei einem Druck von 0,55 mbar der Überschuss p-Hydrochinon ($Kp_{0,55\ mbar}$; 140°C) abdestilliert.
Ausbeute: 330 g Rohprodukt an 3-(4-Oxy-anilino)-9-äthylcarbazol = 110% Ausbeute, bezogen auf eingesetztes 3-Amino-9-äthyl-carbazol, d.h. das Produkt enthält noch ca. 10% p-Hydrochinon.

Wird eine weitere Reinigung angestrebt, so kann das Produkt im Vakuum destilliert werden. $Kp_{0,25\ bis\ 0,55\ mbar}$; 280°C (Badtemperatur 320 bis 340°C).

Wird der Ansatz ohne Jod durchgeführt, so erhält man nach dem Dünnschichtchromatogramm (Fliessmittel Toluol/Essigsäureäthylester 1 : 1) ein weniger reines Produkt; die Elementaranalyse zeigt keine Unterschiede.

Wird statt 3-Amino-9-äthyl-carbazol 3-Amino-9-octyl-carbazol eingesetzt, erhält man in vergleichbarer Weise 3-(Oxy-anilino)-9-octyl-carbazol.

Beispiel 2

450 g 3-Aminocarbazol (2,47 Mol), 500 g p-Hydrochinon (4,55 Mol), 5 g Jod werden unter Stickstoff bei 210 bis 230°C ca. 16 Stunden lang gerührt und das Reaktionswasser abdestilliert. Die Umsetzung wird so lange fortgeführt, bis im Dünnschichtchromatogramm (Fliessmittel Toluol/Essigsäureäthylester 1:1) kein 3-Aminocarbazol mehr nachweisbar ist. Dann wird im Vakuum (0,55 mbar) der Überschuss p-Hydrochinon abdestilliert. Ausbeute: roh 540 g an 3-(4-Oxy-anilino)-carbazol; das Produkt ist genügend rein zur Herstellung des gewünschten Schwefelfarbstoffs. Ist eine weitere Reinigung erwünscht, so kann das Produkt im Vakuum destilliert werden: $Kp_{0,13}$ mbar, ≈310°C.

Wird weniger p-Hydrochinon eingesetzt, so erhält man ein unreineres Produkt, das stumpfere Farbstoffe liefert.

Wird die Umsetzung in Gegenwart von Decalin durchgeführt, so kann dieses Lösungsmittel gleichzeitig als Schleppmittel für das Reaktionswasser dienen.

Beispiel 3

200 g p-Aminophenol (1,83 Mol), 2 g Jod, 200 g 3-Aminocarbazol (1,1 Mol) werden 18 Stunden bei 240°C unter Stickstoff gerührt. Es spaltet sich Ammoniak ab, anschliessend wird der Überschuss p-Aminophenol abdestilliert. Man erhält 260 g 3-(4-Oxy-anilino)-carbazol, das nach dem Dünnschichtchromatogramm mit etwas 4,4'-Dihydroxy-diphenylamin verunreinigt ist. Dieses stört die Herstellung des bekannten Schwefelfarbstoffs jedoch nicht.

Wird anstelle von 3-Amino-carbazol 3-Amino-9-äthyl-carbazol eingesetzt, so erhält man in ähnlich guter Weise 3-(4-Oxy-anilino)-9-äthyl-carbazol.

Beispiel 4

115 g p-Hydrochinon (1,05 Mol), 180 g 3-Aminocarbazol (0,99 Mol), 30 ml Dimethyldiglykol (Diglykoldimethyläther) und 1 g Jod werden 7 Stunden auf 220°C unter Stickstoff erhitzt und über eine kleine Kolonne insgesamt 50 ml abdestilliert (Reaktionswasser + Dimethyldiglykol). Die Ausbeute an 3-(4-Oxy-anilino)-carbazol ist praktisch quantitativ.

Beispiel 5

210 g 3-Aminoäthyl-carbazol (1 Mol), 155 g p-Hydrochinon (1,4 Mol), 2 g Jod, 60 ml Decalin werden 9 Stunden lang auf 210°C (Badtemperatur) unter Stickstoff und unter Rühren erhitzt, wobei über eine kleine Kolonne im Verlauf der Reaktion das Reaktionswasser und das Decalin abdestilliert wurden. Anschliessend wurde der Überschuss an p-Hydrochinon unter vermindertem Druck abdestilliert. Die Ausbeute an 3-(4-Oxy-anilino)-carbazol ist mit 303,5 g praktisch quantitativ.

Beispiel 6

210 g 3-Amino-9-äthyl-carbazol (1 Mol, 176 g p-Hydrochinon (1,6 Mol), 44 g p-Aminophenol (0,4 Mol) und 2 g Jod werden bei 170 bis 180°C unter Stickstoff 14 Stunden lang gerührt, wobei Reaktionswasser und Ammoniak abdestilliert wird. Dann wird bei 0,4 mbar der Überschuss an p-Hydrochinon und p-Aminophenol abdestilliert. Ausbeute 325 g an rohem 3-(4-Oxy-anilino)-9-äthyl-carbazol, das, falls gewünscht, durch Destillation unter vermindertem Druck weiter gereinigt werden kann.

**Patentansprüche**

1. Verfahren zur Herstellung von Derivaten des 3-(4-Oxy-anilino)-carbazols der allgemeinen Formel

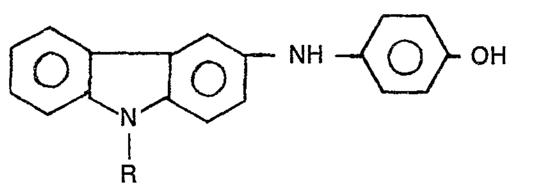

I

worin R = Wasserstoff, Alkyl mit 1 bis 8 C-Atomen, Halogenalkyl mit 1 bis 8 C-Atomen bedeutet, dadurch gekennzeichnet, dass ein 3-Aminocarbazol der allgemeinen Formel

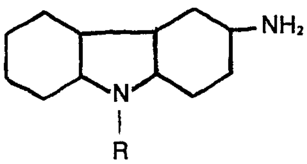

II

mit p-Hydrochinon oder p-Aminophenol oder mit einem Gemisch aus p-Hydrochinon und p-Aminophenol in mindestens stöchiometrischen Mengen unter Ausschluss von Sauerstoff auf Temperaturen von 150 bis 300°C erhitzt wird.

2. Verfahren nach Anspruch 1, dadurch gekennzeichnet, dass auf Temperaturen von 170 bis 230°C erhitzt wird.

3. Verfahren nach den Ansprüchen 1 bis 2, dadurch gekennzeichnet, dass die Reaktion in Gegenwart katalytischer Mengen Jod durchgeführt wird.

4. Verfahren nach Anspruch 3, dadurch gekennzeichnet, dass die Reaktion in Gegenwart von 0,1 bis 4 Gew.-% Jod, bezogen auf die Verbindung der Formel II, durchgeführt wird.

5. Verfahren nach den Ansprüchen 1 bis 4, dadurch gekennzeichnet, dass das p-Aminophenol oder p-Hydrochinon in mindestens der 1,3-fachen stöchiometrischen Menge, vorzugsweise mindestens der 1,6fachen stöchiometrischen Menge, eingesetzt wird.

6. Verfahren nach den Ansprüchen 1 bis 5, dadurch gekennzeichnet, dass die Umsetzung in Gegenwart eines inerten Lösungsmittels, vorzugsweise in Gegenwart von bis zu 15 Gew.-% des inerten Lösungsmittels, bezogen auf das Gesamtgewicht der Reaktanten, durchgeführt wird.

7. Verwendung der nach den Ansprüchen 1 bis 6 hergestellten Leuco-indophenolen der allgemeinen Formel I zur Herstellung von Schwefelfarbstoffen.

## Claims

1. Process of preparing derivatives of the 3--(4-hydroxyanilino)-carbazole of the general formula

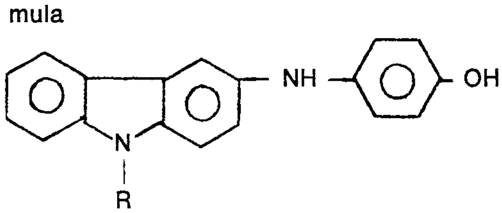

I

wherein R is hydrogen, alkyl having 1 to 8 carbon atoms or halogenalkyl having 1 to 8 carbon atoms, characterised in that a 3-aminocarbazole of the general formula

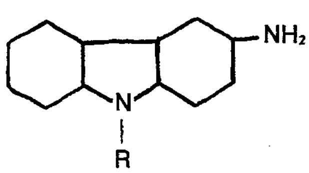

II

is heated with p-hydroquinone or p-aminophenol or with a mixture of p-hydroquinone and p-aminophenol in at least stoichiometric amounts to temperatures of 150 to 300°C with exclusion of oxygen.

2. A process as claimed in claim 1, characterised in that the reactants are heated to temperatures of 170 to 230°C.

3. A process as claimed in claims 1 to 2, characterised in that the reaction is carried out in the presence of catalytic amounts of iodine.

4. A process as claimed in claim 3, characterised in that the reaction is carried out in the presence of 0,1 to 4% by weight of iodine, relative to the compound of formula II.

5. A process as claimed in claims 1 to 4, characterised in that the p-aminophenol or p-hydroquinone is employed in an amount of at least 1,3 times the stoichiometric amount, preferably at least 1,6 times the stoichiometric amount.

6. A process as claimed in claims 1 to 5, characterised in that the reaction is carried out in the presence of an inert solvent, preferably in the presence of up to 15% by weight of the inert solvent, relative to the total weight of the reactants.

7. Use of the leuco-indophenols of the general formula I prepared according to claims 1 to 6 for the preparation of sulphur dyestuffs.

## Revendications

1. Procédé de préparation de dérivés de 1'(hydroxy-4-anilino)-3-carbazole répondant à la formule générale I

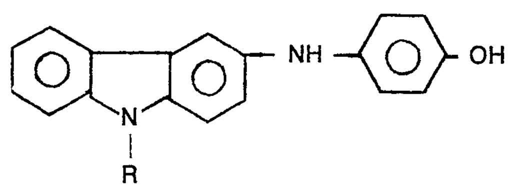

I

dans laquelle R représente l'hydrogène, un radical alkyle contenant de 1 à 8 atomes de carbone ou un radical halogéno-alkyle contenant de 1 à 8 atomes de carbone, procédé caractérisé en ce qu'on chauffe un amino-3-carbazole de formule générale II

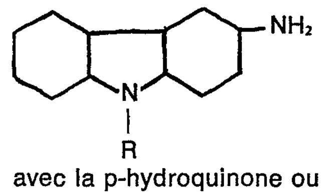

II

avec la p-hydroquinone ou le p-aminophénol, ou avec un mélange de p-hydroquinone et de p--aminophénol, en des quantités au moins stoechiométriques, à l'abri de l'oxygène, à des températures de 150 à 300°C.

2. Procédé selon la revendication 1 caractérisé en ce qu'on chauffe à des températures de 170 à 230°C.

3. Procédé selon l'une des revendications 1 et 2 caratérisé en ce qu'on effectue la réaction en présence de quantités catalytiques d'iode.

4. Procédé selon la revendication 3 caractérisé en ce qu'on effectue la réaction en présence de 0,1 à 4% en poids d'iode par rapport au composé de formule II.

5. Procédé selon l'une quelconque des revendications 1 à 4 caractérisé en ce qu'on met en jeu le p-aminophénol ou la p-hydroquinone en une quantité représentant au moins 1,3 fois la quantité stoechiométrique, de préférence au moins 1,6 fois.

6. Procédé selon l'une quelconque des revendications 1 à 5 caractérisé en ce qu'on effectue la réaction en présence d'un solvant inerte, utilisé de préférence en une quantité allant jusqu'à 15% en poids par rapport au poids total des corps prenant part à la réaction.

7. Application des leuco-indophénols de formule générale I préparés selon l'une quelconque des revendications 1 à 6 à la préparation de colorants au soufre.